# EUROPEAN PATENT APPLICATION

(11) **EP 3 556 843 A1**
(43) Date of publication of application: **23.10.2019**
(21) Application number: 18168285.7
(22) Date of filing: 19.04.2018
(51) Int. Cl.: C12M 1/26, C12M 1/34, C12M 1/36, G01N 1/02

(54) **HIGH-PRECISION RAPID SAMPLING DEVICE**

(71) Applicant: Technische Universität Graz, 8010 Graz (AT)
(72) Inventor: KLIMACEK, Mario, 8045 Graz (AT); SCHARFETTER, Hermann, 8045 Graz (AT); HILBER, Dominik, 8380 Jennersdorf (AT)
(74) Representative: Gassner, Birgitta

(57) **Abstract**

The invention relates to an automated sensor-controlled rapid sampling device comprising a mass sensor and a pressure sensor for accurately determining the mass of a sampled fluid.

## Description

### Field of the Invention

The present invention relates to the field of high-precision sampling devices.

### Background Art

Analysis of intracellular metabolites (metabolomics) represents a key technique applied in human and biosciences for cell phenotyping and identification of health-associated biomarkers and metabolic bottlenecks, respectively. This technique provides therefore the ultimate basis for future novel diagnostics, pharmaceuticals, and microbial cell factories.

The metabolite profile of a cell is a direct representation of the cellular phenotype and its active reaction network. Consequently, metabolite levels are highly dependent on the cultivation conditions employed and thus extremely sensitive to changes in process conditions. It is expected that changes in the metabolite profile due to changes in the cellular environment take place within the sub-second time range (< 0.2 s). Preparation of a truly representative intracellular metabolite sample is a complex multi-step procedure that involves sampling, quenching of cellular metabolism and extraction of intracellular metabolites. Obtained metabolite extracts are subjected to metabolite analysis by mass spectrometry- or NMR-based analytics. Unbiased record of metabolite pools requires that each step is optimized with respect to metabolite conservation. To enable scientifically solid interpretations intracellular metabolite data are presented in molar units per g dry cells or a defined number of cells. As both molarity of intracellular metabolites and amount of cells are dependent on the sample volume knowledge of the exact volume withdrawn from the cultivation vessel is required.

Withdrawing a sample from the cultivation broth is the first step in the workflow of sample preparation. This step comprises rapid withdrawal of a predefined amount of cell broth from a cultivation vessel, which is followed by the immediate inactivation of the cellular metabolism both without altering the metabolite profile of the cells. In addition to the preservation of metabolite pools knowledge of the exact sample volume is essential to enable calculation of reliable intracellular metabolite data. In current approaches a pre-specified volume of cell broth is transferred either manually by using a pipette or via valve-controlled manual or automated sampling devices into an appropriate sample container containing a suitable quenching solution. However manual sample transfer by pipetting requires opening of the reaction vessel and provides therefore a substantial source of contamination of the culture and in case of anaerobic cultivation, in addition, by air oxygen thereby altering the physiological state of the cells to be investigated. Furthermore the long sampling time required for manual sampling limits on one hand its applicability for studies targeting metabolite dynamics and on the other hand can affect the cellular state as oxygen may become limiting for cells sitting in the pipette tip. To overcome these limitations a series of sampling devices with improved sampling time has been developed in the past, which are connected to the cultivation vessel thereby permitting sampling from a closed reaction vessel. Depending on the sampling device different volumes can be addressed ranging from 1 mL to 10 mL. To the best of our knowledge there is no instrument commercially available today. Another disadvantage of most published and patented instruments is that they are applicable only to a certain type of cultivation vessel and many of them even require special installations to be built in cultivation vessels which typically ends up with irreversibly altered vessels. Consequently these sampling devices are not compatible with other cultivation vessels or vessel types which greatly reduce their general applicability in the lab.

However, the greatest disadvantage of all hitherto known sampling devices is that the exact sample volume is not known and can only be assumed. According to the procedures of the state of the art the volume to be sampled is calibrated externally under defined conditions and it is assumed that the conditions are maintained during the sampling process and not altered. Gravimetrical determination of the sample volume subsequent to the sampling event is not an option because metabolite samples must be processed as quickly as possible to preserve the metabolite state. With the exception of one device with which cell broth is continuously sampled at a constant flow by using a peristaltic pump (note this instrument was especially developed for carrying out pulse experiments), sampling is driven by the overpressure and hydrostatic pressure present in the cultivation vessel. To increase the pressure difference between cultivation vessel and sample container, the sample container can be evacuated. The flow rate of the cell broth through the tubing of the sampling device is largely determined by this pressure difference. In addition fluid properties such as viscosity affect the flow rate and therefore the sample volume. Consequently changes in cultivation volume (due to fed batch operation and sampling; alters hydrostatic pressure;) and overpressure in the reaction vessel (by cell growth, increased aeration and CO₂ production) as well as differences in the negative pressure in the sample container (leakage, sample-to-sample variation) affect significantly the sampled volume. Another source of variation in the sampled volume represents co-sampling of gas bubbles (air, nitrogen). As a consequence differences in sample volumes provoked by these changing process conditions are not recognized by current sampling devices and resultant metabolite concentrations are therefore afflicted with unknown uncertainty which eventually leads to false interpretation of results. Furthermore as intracellular metabolite analysis is extremely expensive knowledge of the exact volume of sampled cell broth is therefore, from an economic point of view, essential to increase the number of reliable metabolite samples.

Rapid sampling devices may be roughly divided into two groups depending on the site at which the sampling tube is connected to the cultivation vessel. Sampling devices of the first group are connected via special installation at the bottom or the side wall of a bioreactor while those of the second group are connected at the head plate of a bioreactor or at the vessel cap of a flask. Different to instruments of the first group which are restricted to cultivation vessels made of metal members of the second group are also applicable to cultivation vessels made of glass.

In order to transfer a cell broth sample from the cultivation vessel to the sample container either a high-precision peristaltic pump was used to maintain constant fluid flow, or the transfer is driven by the pressure difference between cultivation vessel and sample container. For instruments of the first group hydrostatic pressure formed by the working volume and over-pressuring of the cultivation vessel has been applied. To increase the pressure difference the sample container can be evacuated in addition. For devices of the second group application of an overpressure to the cultivation vessel or of a specialized vacuum-sealed sample container has been shown to be functional. Vacuum-sealed sample containers are of limited applicability because (i) pressure difference is difficult to be reproduced and (ii) specially constructed sample containers are required which increases the costs per sample significantly.

Further, dead volumes should be avoided or at least minimized because of potential carry-over of sample content from one to the next sample and over time being a source for deposits and microbial contamination. Thus, sampling devices with little to no dead volumes were developed. Alternatively devices were reported which remove stagnant fluid by evacuation or flushing with cell broth, sterile air, and disinfection solution. In addition to dead volumes laminar flow can lead to significant carry-over due to axial dispersion.

An important prerequisite for sampling devices employed in the context of intracellular metabolite analysis is that the residence time is close to or even below 1 sec. This includes the transfer as well as the stopping of the cellular metabolism.

In the past a series of different rapid sampling devices were developed in the context of rapid sampling and immediate inactivation of cellular metabolism. EP1508791 discloses a device for automated bioreactor sampling. The device comprises a sampler, a tube connecting the sampler with the reactor and a pump for pumping fluids from the reactor to the sampler. Between or during the individual sampling steps at least parts of the tubing are flushed with gas.

Therefore, there is still the need for a sampling device with accurate mass determination within a short time frame. As mass is directly related to the volume via the density the sample volume withdrawn from the cultivation vessel can be directly addressed.

### Summary of invention

It is the objective of the invention to provide a sampling device, which is able to withdraw samples from reaction vessels selectively with respect to carry-over and with accurate mass determination and within short time.

The objective is solved by the subject matter as claimed and further described herein.

One embodiment of the invention relates to a rapid sampling device for a bioreactor comprising a sampling mean (I), a pump unit (II), a valve unit (III), and a docking unit (IV), wherein the docking unit comprises a docking port (4) equipped with a pressure sensor (6) and a mass sensor (5).

A further embodiment of the invention relates to the device as described herein, wherein the docking port (4) further comprises a sample holder (73) and a sample container (7).

A further embodiment of the invention relates to the device as described herein, wherein the sample holder (73) comprises a polymer layer (79).

A further embodiment of the invention relates to the device as described herein, wherein the docking unit (IV) further comprises a vibration damper (78).

A further embodiment of the invention relates to the device as described herein, wherein the docking unit (IV) further comprises a control unit (76).

A further embodiment of the invention relates to the device as described herein, wherein the docking unit (IV) further comprises fixation means (74).

A further embodiment of the invention relates to the device as described herein, wherein the valve unit (III) further comprises a vacuum container (2) and a waste container (2').

A further embodiment of the invention relates to the device as described herein, wherein the valve unit (III) is a 5-valve unit.

A further embodiment of the invention relates to the device as described herein, wherein the sampling mean (1) is a tube.

A further embodiment of the invention relates to the device as described herein, wherein the tube (1) is made of a highly hydrophobic material, preferably of fluorinated ethylene propylene copolymer (FEP).

One embodiment of the invention relates to a process for reliable determination of the volume of a sample withdrawn from a bioreactor, wherein the mass of the withdrawn sample is determined by the device as described herein and subsequently the volume calculated.

One embodiment of the invention relates to the use of the device as described herein in the field of metabolomics, proteomics, microbial fermentation, enzyme production, organic or inorganic synthesis.

### Brief description of drawings

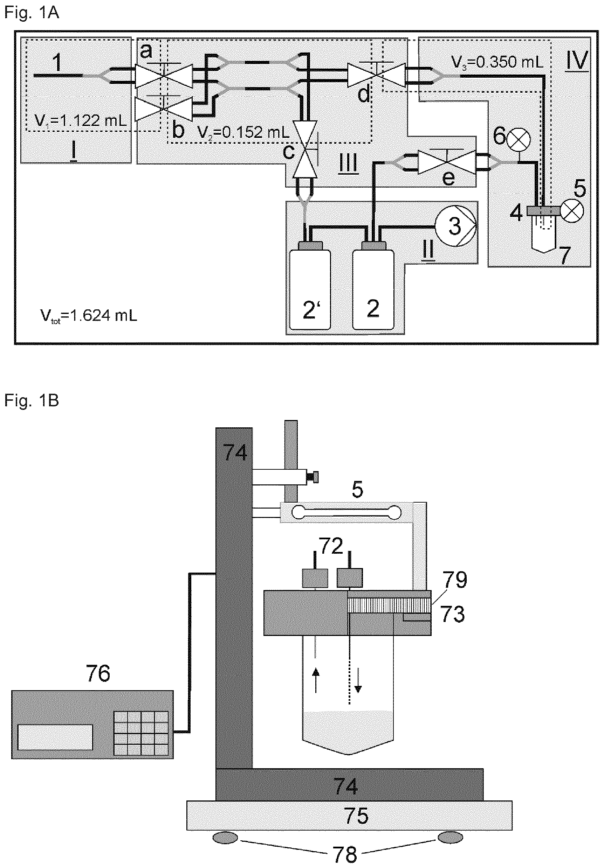

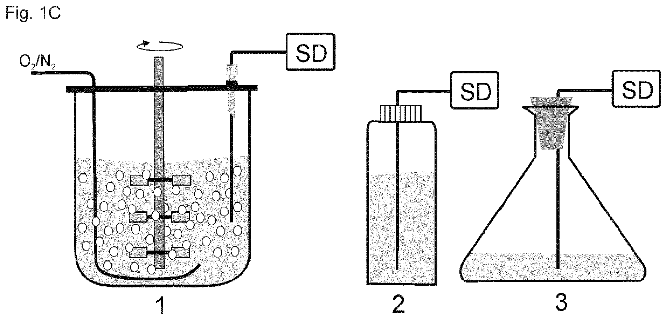

Fig. 1 depicts organization of the 5-valve unit (Panel A), a scheme of the sampling unit (Panel B) and examples of applicable cultivation vessels (Panel C

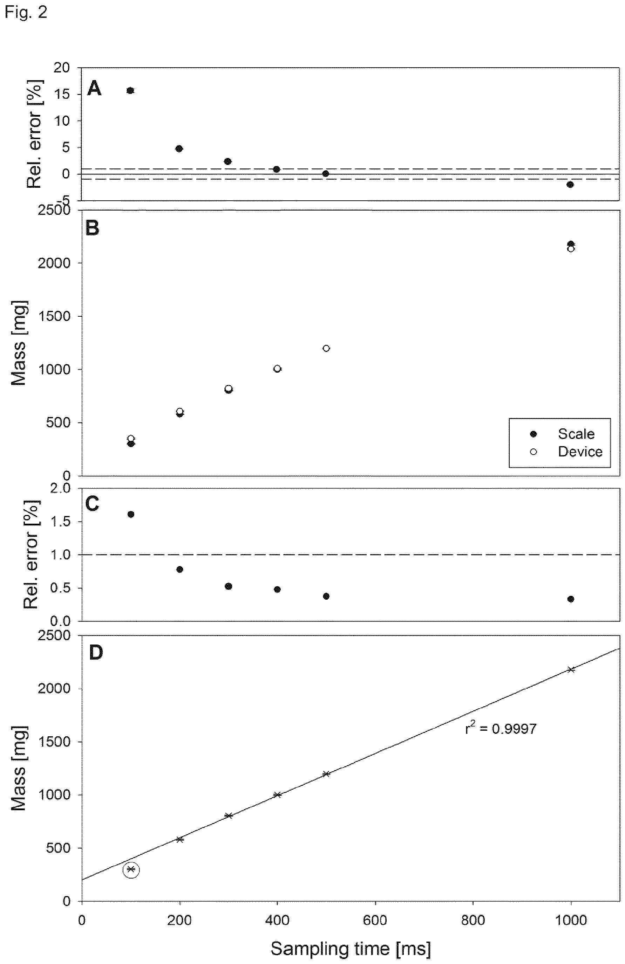

Fig. 2 shows the correlation between sampling time and mass of transferred fluid. Panel A: Mean relative deviation between masses obtained by the device and scale. Values were calculated by the relationship (m_{device}-m_{scale})*100/m_{scale} in which m indicates the average of 10 individual samples. Dashed and solid lines indicate the ±1% limits and the 0% reference for the relative deviations, respectively. Panel B: Comparison of masses determined by an analytical scale (closed symbols) and device (open symbols). Panel C: Reproducibility error of masses measured by the device in dependence of the sampling time. Values, shown as rel. errors, were calculated by the relationship standard deviation * 100 / average; n=10. Panel D: Mass of transferred sample in dependence of the sampling time. Averages and corresponding standard deviations are shown. High linearity (solid line) was observed for masses between 200 - 1000 ms sampling times (mass = Sampling time * 1.98 ± 0.02 + 201 ± 12).

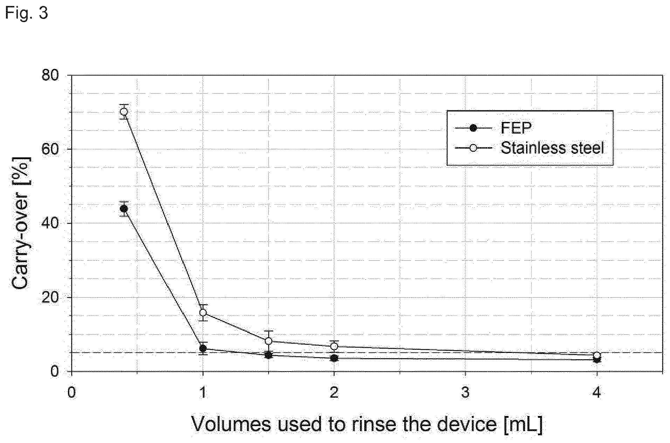

Fig. 3 shows the carry-over between two subsequent samples in dependence of the volume used to rinse the tubing as well as of the type of sample tube applied (FEP (closed symbols) or stainless steel (open symbols)). Carry-over by 5% is indicated by a dashed line.

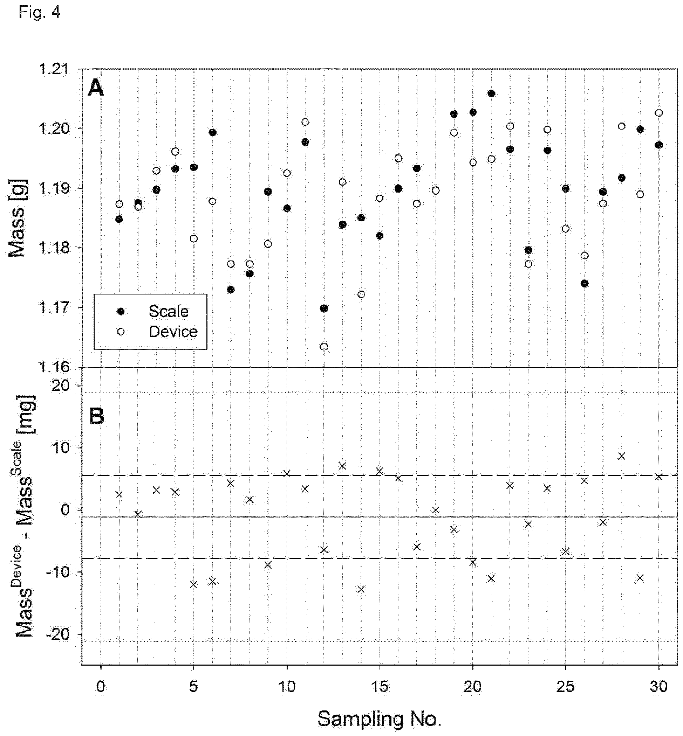

Fig. 4 shows the accuracy of the rapid sampling device determined by sampling water from an Erlenmeyer flask. Panel A shows masses determined by the analytical scale (full symbols) and the rapid sampling device (open symbols). Panel B indicates corresponding mass differences and the average difference (solid line) is shown together with variation of ± one standard deviation (dashed lines) and ± three standard deviations (dotted lines).

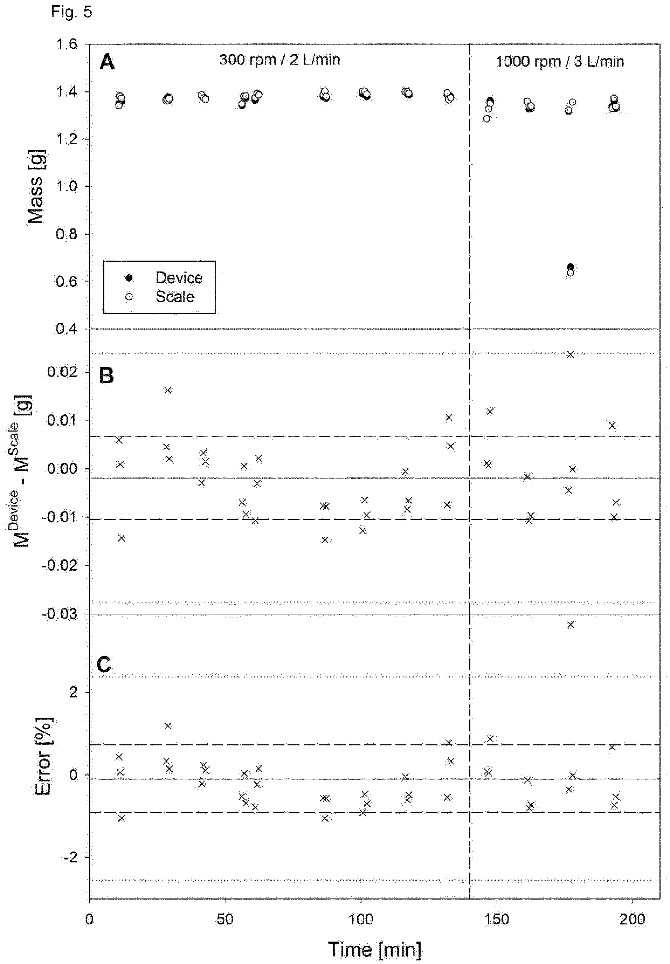

Fig. 5 shows the accuracy of masses determined by the rapid sampling device transferring cell broth from a bioreactor cultivating *S. cerevisiae* in mineral medium, pH 5.2 under aerobic conditions into a cold methanol solution (10 mL). Note aeration and stirring conditions were changed as indicated by vertical dashed lines in Panel A-C after 140 min. Panel A shows masses determined by the rapid sampling device (full symbols) and an analytical scale (open symbols). Panel B displays corresponding residuals between masses obtained from the device and scale while in Panel C residuals were related to the average mass calculated from both device and scale. Solid, dashed and dotted lines represent the average, ±1 standard deviation, ±3 standard deviation, respectively.

### Description of embodiments

The present invention relates to the development of an automated rapid sampling device with which a predefined sample volume e.g., 1 mL or 2 mL which are typical volumes used in current metabolite analysis, of cell broth can be selectively (little to no carry-over from one to the next sample) withdrawn from any reaction vessel and transferred into a flask containing an appropriate quenching solution and mixed with this solution rapidly in 1.4 or 1.1 sec. The mass of each sample is measured by a mass sensor. Time point of sampling and the mass of each sample is recorded and data can be transferred to a standard computer. Finally the exact volume of the withdrawn sample is determined by including the density.

As used herein, the term "sample" may be used to refer to a growth (reaction) medium, a mixture of cells and the growth medium or mixture of a (bio)catalyst and the reaction medium.

The objective of this invention is to provide a sampling device which is capable of measuring the exact mass (and therefore the volume from known density) of each sample withdrawn from a cultivation vessel. The device is compatible with all common lab-scale cultivation vessels. One exemplary embodiment is depicted in Fig. 1, panel C. Furthermore, the sampling device enables a low residence time and shows minimal carry-over.

The cultivation vessel may be a simple shaken or stirred flask or a fermenter (bioreactor). The cultivation volume may be in the range of 10 mL to 5 L, such as a shake flask (bottle), or in the range of 1-1000 L, such as a laboratory fermenter, or even in the range of 1 to over 100 m³, such as industrial production reactors. The term cultivation vessel may also refer to reaction vessels (lab, pilot or industrial scale) used in enzymatic, inorganic and organic syntheses.

An exemplary set-up of the sampling device is shown in Figure 1, panels A and B. The sampling device is composed of four functional units, namely a sampling unit (I), a pump unit (II), a valve unit (III), and a docking unit (IV).

The sampling unit (I) comprises sampling means. The sampling means may be sampling tubes or sampling lines. The sampling means may be made of any suitable material which may further be suitable for sterilization processes, such as for example, metal, stainless steel, polymeric material or glass. Preferably the sampling mean is made of fluorinated ethylene propylene (FEP) (or of any material displaying similar properties), a copolymer of hexafluoropropylene and tetrafluoroethylene. Sampling means made of FEP have the advantage of low friction and non-reactivity. Carry-over contamination of one sample to another is problematic if strategies to manage accidental transfer of sample residues are not implemented. Due to the advantageous properties of FEP carry-over of residues of one sample into a subsequent sample is highly reduced. This is accompanied by the advantage of little loss of culture broth due to reduced flushing procedures.

In one embodiment of the invention the sampling tube is made of fluorinated ethylene propylene (FEP) instead of stainless steel which decreases significantly the axial dispersion and in consequence carry-over of sample content.

The sampling mean can be fitted aseptically into the cultivation vessel.

The pump unit (II) provides for pumping of fluids with a controlled pressure of said fluids. One embodiment of the invention relates to a pump unit (II), wherein the pump unit is a vacuum pump (3). The pump unit (II) further comprises a waste container (2') and a vacuum container (2).

The valve unit (III) may comprise 3, 4, 5, 6 or more valves. In one embodiment of the invention the valve unit comprises 5 valves.

Tubing in the 5-valve module contains two identical, parallel branches to increase the fluid flow (decrease of the residence time) by decreasing flow resistance. The 5-valve module is designed to enable withdrawal of fluid from the bioreactor at different predefined volumes covering a range of 0.4 mL - 300 mL (corresponds to the detection range of the used mass sensor).

The docking unit (IV) comprises the docking port which is equipped with a pressure (6) and a mass sensor (5). This permits recording of the mass of each sample withdrawn. Sample-to-sample variations are recognized and can be therefore considered in subsequent analytics. As an important consequence and in contrast to hitherto known instruments the novel device is not dependent on the type of cultivation or reaction vessel applied and is thus highly versatile in lab-scale cultivation and synthesis experiments.

The pressure and the mass sensor may be any reasonable device which meets the specifications of reproducibility and resolution. For the mass sensor the resolution is determined by the ratio between maximum allowable mass error and maximum expected total mass of the filled sampling vessel.

The docking port comprises fixation means (74) for the sample container (7). In one embodiment of the invention the fixation means are provided as housing or as holding fixture. The fixation means (74) may comprise the mass sensor (5), a sample holder (73) which may comprise a polymer layer (79). The fixation means (74) may rest on a plate (75) with elastic vibration dampers or alternative means to quell vibrations (78).

The docking port further comprises a control unit (76). The control unit comprises a power supply, a display, an interface and navigation keys. The control unit may be arranged to control the sampling device and to store the time, pressure and the mass.

Recording of the pressure permits identification of system leakage and serves therefore as quality control.

A detailed description of the characteristics of the developed device is given in Table 1.

**Table 1 - Characteristics of the developed rapid sampling device**

| Special features | Mass of each sample withdrawn is measured at high accuracy |
|---|---|
| | Mass and time of sampling is recorded and can be transferred to a standard computer |
| Automated valve-controlled sampling driven by vacuum | ca. 10 kPa |
| Sample volume | Variable: 0.4 - 300 mL |
| Total volume, instrument | 1.62 mL |
| Rate of fluid flow | 2.0 mL/s |
| Residence time of sample | Dependent on the sample volume: 0.9 - 1.4 s for 4.0 - 0.4 mL |
| Accuracy on mass | < 0.8% |
| System conditioning | 1.5 mL of reaction broth |
| Carry-over of sample content | < 5% |
| Sampling frequency | ca. 25 sec / sample |
| Mixing time | 1.0 ± 0.1 sec for a 1.2 mL sample |
| Sterility | Sterile connection possible |
| Formation of aerosols | No |
| Formation of ice crystals | No |
| Sample container | 15 mL or 50 mL Corning /Falcon tubes |

The present sampling device is specifically suited for sampling of a known sample volume (mass) directly into a fluid. The fluid may be a quenching fluid. "Quenching" or "quenched" means that processes (these may relate to metabolic activity in cells, (bio)catalyzed inorganic or organic reactions) within the sample (cells) are stopped. The quenching solution may comprise solutions or solvents suitable to quench/stop cellular metabolism or chemical reactions.

Sampling according to the method as described herein may be carried out without opening of the reaction vessel.

The rapid sampling device may especially be suited for metabolomics but also proteomics, microbial fermentation, enzyme / organic / inorganic synthesis.

Other characteristics of the rapid sampling device:
The sampling tube can be fitted aseptically to every lab-scale reaction vessel and permits withdrawal of cell broth by maintaining process conditions. A polymer seal (KLASSE 4 - TURBOSIL was used in the present device but other polymers with similar properties may be applicable) used in the docking unit serves as a hermetic septum for the hypodermic needles and ensures air-tight connection between the vacuum pump the 5-valve module and the sample container (docking unit).

Carry-over of sample contents between subsequent samples is < 5% due to (i) flushing both the sampling tube and tubing of the 5-valve module with 1.5 mL of cell broth, (ii) flushing the tubing of the 5-valve module with air and (iii) using a sampling tube made of a highly hydrophobic material (FEP) instead of stainless steel which has been commonly used in the past.

To minimize the transfer of mechanical vibrations to the mass sensor the sampling unit is placed on a heavy stone slab resting on two elastic dampers. In addition the 5-valve module is arranged such that valve operation does not interfere with mass measurements

At the data acquisition (DAQ) steps an average mass of the empty (step 4, DAQ 1) or filled (step 15, DAQ 2) sample container is determined. To this end 10 individual measurements are carried out by the mass sensor and after excluding potential outliers an average is calculated from the remaining values. After calculating the difference the resultant value is converted into an accurate mass value by multiplying with the correction factor obtained from the calibration procedure.

Quality control routines were developed to guarantee the required accuracy of the mass measurements (by system calibration) and to identify leakage in the system by using a pressure sensor

The timing protocol of the 5-valve module can be modified through a graphical PC-software and transferred to the device by USB. When injected into the sample container the sample forms a well-confined narrow jet and mixes immediately with the solution.

In one embodiment of the invention the instrument works as follows:
- The instrument is calibrated;
- The sampling tube is connected (if required aseptically) to the reaction vessel and the power supply and vacuum pump are switched on;
- Initialize the instrument for sampling by selecting a pre-defined timing protocol (step 1 of the timing protocol stated in Table 1)
- Attach a sample container to the docking port;
- Press start (step 2 of the timing protocol);
- After 3000 ms the programmed timing protocol is carried out automatically;
- After completion of the sampling the sample container is dismounted;
- Mass, time and pressure are automatically stored by the instrument.

A representative timing protocol for sampling and DAQ is shown in Table 2 (valve numbers as used in Fig. 1, Panel A).

**Table 2 - Timing protocol for one cycle of the 5-valve module and data acquisition DAQ**

| Step No | Action | Valve a | Valve b | Valve c | Valve d | Valve e | Mass Sensor | Pressure sensor | Time [ms] | Remarks |
|---|---|---|---|---|---|---|---|---|---|---|
| 1 | Initiation | Closed | Closed | Closed | Closed | Open | Inactive | Active | | Ready for docking |
| 2 | Docking | Closed | Closed | Closed | Closed | Open | Inactive | Active | | Attach tube to docking unit and press initiation button |
| 3 | Wait | Closed | Closed | Open | Closed | Open | Inactive | Inactive | 3000 | Time required until vacuum is ready and docking vibrations have decayed below threshold to avoid bias of the readout |
| 4 | DAQ 1 | Closed | Closed | Open | Closed | Open | Active | Inactive | 1500 | Mass measurement of attached empty sample container |
| | **Start of sampling** | | | | | | | | | |
| 5 | Rinsing | Open | Closed | Open | Closed | Open | Inactive | Inactive | 750 | Rinsing the sampling tube and tubing of the 5-valve module with fluid from the bio (reaction) reactor |
| 6 | Wait | Closed | Closed | Open | Closed | Open | Inactive | Inactive | 50 | Valve switch |
| 7 | Flushing I | Closed | Open | Open | Closed | Open | Inactive | Inactive | 200 | Flushing fluid present in the tubing of the 5-valve module into the waste container with air |
| 8 | Wait | Closed | Closed | Open | Closed | Open | Inactive | Inactive | 50 | Valve switch |
| 9 | Wait | Closed | Closed | Closed | Closed | Open | Inactive | Inactive | 50 | Valve switch |
| 10 | Wait | Open | Closed | Closed | Closed | Open | Inactive | Inactive | 50 | Valve switch |
| 11 | Sampling | Open | Closed | Closed | Open | Open | Inactive | Inactive | 200-1.5x10⁵ | Sample transfer; time depends on the sample volume |
| 12 | Wait | Closed | Closed | Closed | Open | Open | Inactive | Inactive | 50 | Valve switch |
| 13 | Flushing II | Closed | Open | Closed | Open | Open | Inactive | Inactive | 200 | Flushing fluid present in the tubing of the 5-valve module and the docking unit into the sample container with air |
| 14 | Wait | Closed | Open | Closed | Closed | Open | Inactive | Inactive | 1000 | Valve switch |
| 15 | DAQ 2 | Closed | Open | Open | Closed | Open | Active | Active | 1500 | Mass measurement of attached sample container + sample |
| | **End of sampling** | | | | | | | | | |
| 16 | Wait | Closed | Closed | Open | Closed | Open | Inactive | Inactive | 50 | Valve switch |
| 17 | Wait | Closed | Closed | Closed | Closed | Open | Inactive | Inactive | 50 | Valve switch |
| 18 | End | Closed | Closed | Closed | Closed | Closed | Inactive | Inactive | | End of cycle |

Based on the timing protocol shown in Table 2, assuming that the flow of liquid through the tubing of the device is 2.0 mL/s (see Fig. 2D) and that the time needed for the sample to leave the needle tip and enter the quenching solution is, as was estimated by video analysis, -35 ms residence times of 1.40 s and 1.14 s can be estimated for sampling of 1 mL and 2 mL, respectively, both are close to the widely accepted value of 1 sec.

Aside from its primary use in withdrawing samples subjected to metabolomics analysis the instrument is suitable for all applications in which either knowledge of the exact volume of the transferred sample is required and/or the reaction vessel is not allowed to be opened.

It is suggested that in addition to intracellular metabolite analysis the instrument may find application in proteome analysis as well as for enzyme/organic/ inorganic synthesis.

Furthermore the instrument could be as well used to withdraw fermentation samples for routine analytics (cell density, product analysis). Using this instrument would lead to a reduction of total sample volume by 90%. Typically 10-15 mL of fermentation broth are lost during sampling due to conditioning of sample tubes/pipes. With the developed device only 1.5 mL are removed in addition to the sample Examples

The Examples which follow are set forth to aid in the understanding of the invention but are not intended to, and should not be construed to limit the scope of the invention in any way. The Examples do not include detailed descriptions of conventional methods. Such methods are well known to those of ordinary skill in the art.

### Example 1 - Correlation between sample volume and sampling time

Water was transferred to an empty 50 mL tube at different sampling times (step 11 in Table 1) which were varied from 150 ms to 1000 ms. Transfers of liquid were repeated ten times. The mass of the sampled liquid was determined by the mass sensor of the device and, for comparison, with an analytical scale (Kern 770, reproducibility 0.1 mg, linearity 0.2 mg, readability 0.1 mg). The device was calibrated at a sampling time of 500 ms. Results are shown in Figure 2.

From Fig. 2, it can be seen that the device accurately (Panels A and B) and reproducibly (Panels C and D) transferred fluid and measured masses at the sampling time used for system calibration (500 ms). Longer or shorter sampling times resulted in apparently lower or higher masses, respectively measured by the device as compared to those obtained by the scale (Panels A and B). The deviation increased with increasing difference between applied sampling time and sampling time selected for calibration. The results imply that calibration of the instrument should always be done at a sampling time corresponding to the desired sampling volume in a particular experiment. Currently the accuracy of mass data acquisition during DAQ 2 (step 15) is limited by the poorly damped mechanical oscillations of the mass sensor which are induced by loading and unloading. A significant part of the error is due to incomplete settling at the time of DAQ 2. An increase of the accuracy could be achieved in the future by fitting a model of the mechanical ringing process to the data. This may also render unnecessary the calibration procedure.

Sample-to-sample variation of the transferred sample volumes was below 0.8% for all addressed sampling times except for 150 ms (Panel C). Samples withdrawn with 150 ms showed a variation of 1.6 %. The device showed a linear relationship between measured sample mass and sampling time for sampling times between 200 and 1000 ms (Panel D). The obtained slope value (2.0 g/s = 2 mL/s, assuming a density of 1 for water) provides an estimate for the fluid flow through the tubing. Results obtained by this study thus indicate that sample volumes ≥ 400 µL can be withdrawn with high precision and reproducibility. We did not determine the upper bound but the mass range of the mass sensor used may be applicable to samples up to 300 g of fluid.

### Example 2 - Characterization of the carry-over

### Solutions used:

Solution 1: Potassium phosphate buffer: 100 mM, pH 7.0
Solution 2: -60 mg of Congored solved in 1 L of Solution 1 (absorption at 485 nm -1.5)

### Procedure applied:

A 250 mL Erlenmeyer flask was filled with Solution 1 and the sampling tube of the rapid sampling device fitted in appropriately. Three samples were then withdrawn by activating the timing protocol according to Table 2. Thereafter Solution 1 was replaced by Solution 2 and again three samples were withdrawn. This procedure was repeated 3 times. Empty 50 mL falcon tubes were used as sample containers. The absorbance of samples obtained from the rapid sampling device was measured at 485 nm. As a reference for the sampled aliquots two 1 mL samples were withdrawn by pipetting from the respective solution present in the Erlenmeyer flask. Carry-over of sample content was determined between the last and first sample of each sampling series as a function of the duration of rinsing (step 5 in Table 2) which was varied between 200 ms and 2000 ms (1000 ms correspond to 2 mL). The experiment was made for two different materials of the sampling tube, i.e. stainless steel and FEP. The length of both tubes was 275 mm and the inner diameters were 1.8 mm and 1.58 mm for the stainless steel and the FEP tube, respectively. Results shown in Fig. 3 revealed that both rinsing volumes (carry-over decreased with increasing volumes) as well as the type of material (lower carry-over when FEP was used instead of stainless steel) used as sampling tube significantly affected the carry-over. A carry-over < 5 % could be obtained by applying 1.5 mL for rinsing the tubing and a sampling tube made of FEP.

### Example 3 - Determination of variability of withdrawn volumes

To determine the variability of sampled volumes, masses analyzed by the mass sensor were compared to those obtained by weighing (an analytical scale with a resolution of 0.1 mg was used). In the first setup water was sampled 30-times from a 250 mL Erlenmeyer flask. Results are summarized in Fig. 4 (Panel A and B). Masses obtained by the device were statistically not different from those measured by the scale by -1.1 ± 6.7 mg (highest absolute difference: 12.8 mg) at a total averaged mass of 1189.1 mg corresponding to a relative error of 0.1 ± 0.6%. The device sampled water reproducibly with a precision of 1188.5 ± 9.5 mg (0.8% relative error) which is close to or even better than the uncertainties reported for commercial high precision single channel volume adjustable pipettes (e.g. from Eppendorf: model 100 - 1000 µL: 1000 ± 6 µL; model 1-10 mL: 1000 ± 30 µL; 3%, Ref. Eppendorf SOP). As the device is designed for volumes in the range of 0.5 - 300 ml the device's reproducibility should be compared with that of the 1-10 ml pipette.

In the second setup *Saccharomyces cerevisiae* 7D cells were cultivated at 30°C in a stirred 2 L bioreactor (Labfors 2, Infors, Bottmingen, Switzerland) containing mineral medium. The pH value was held at a constant level of 5.2 by PID controlled addition of acid or base. Two conditions for agitation and air sparging were chosen, 300 rpm / 2 L/min and 1000 rpm / 3 L/min. Aeration parameters were chosen to simulate medium and high oxygen demand conditions typically applied to microbial cultivations. Cell broth was withdrawn over time and, to simulate metabolomics sample, transferred into 10 mL of a cold methanol solution (∼70°C, stored on dry ice). Recorded masses by the rapid sampling device were compared to masses obtained from an analytical scale. Results are shown in Fig. 5 (Panels A-C).

Masses sampled by the rapid sampling device in the first phase of fermentation differed from those measured by an analytical scale by -2.8 ± 7.6 mg. The deviation was slightly higher in the second phase, namely -0.1 ± 10.8 mg (Panel B). This difference was predominantly due to one sample at time point 177 min showing a larger residual by +3 standard deviations and a relative error larger than 3 standard deviations (see Figure 5 Panels B and C). This large deviation from the rest of the data was a consequence of the lower sample volume withdrawn from the fermenter which lies outside of the calibration range of the instrument. It is an extremely important feature of this device that such errors can be immediately and easily recognized and taken into account in further analysis. The sample volume may fluctuate from sample to sample due to (i) changes in process conditions (pressure, working volume) and (ii) co-sampling of gas as it was the case in this example. If a constant sample volume would have been assumed as it is commonly done for current sampling devices relative errors of 1.6 - -2.6 % (phase 1) and even larger errors of 8.8 - -52% (phase 2) would have been passed unnoticed under typical aeration conditions.

## Claims

1. A rapid sampling device for a bioreactor comprising a sampling mean (I), a pump unit (II), a valve unit (III), and a docking unit (IV), wherein the docking unit comprises a docking port (4) equipped with a pressure sensor (6) and a mass sensor (5).

2. The device according to claim 1, wherein the docking port (4) further comprises a sample holder (73) and a sample container (7).

3. The device according to claim 2, wherein the sample holder (73) comprises a polymer layer (79).

4. The device according to any one of claim 1 or 3, wherein the docking unit (IV) further comprises vibration damper (78).

5. The device according to any one of claims 1 to 4, wherein the docking unit (IV) further comprises a control unit (76).

6. The device according to any one of claims 1 to 5, wherein the docking unit (IV) further comprises fixation means (74).

7. The device according to any one of claims 1 to 6, wherein the valve unit (III) further comprises a vacuum container (2) and a waste container (2').

8. The device according to any one of claims 1 to 7, wherein the valve unit (III) is a 5-valve unit.

9. The device according to any one of claims 1 to 8, wherein the sampling mean (1) is a tube.

10. The device according to claim 9, wherein the sampling tube is made of a highly hydrophobic material, preferably of fluorinated ethylene propylene (FEP).

11. A process for reliably determination of the volume of a sample withdrawn from a bioreactor, wherein the mass of the withdrawn sample is determined by the device according to any one of claims 1 to 10 and subsequently the volume calculated.

12. Use of the device according to any one of claims 1 to 10 in the field of metabolomics, proteomics, microbial fermentation, enzyme production, enzymatic, organic or inorganic synthesis.
